(19) Europäisches Patentamt
European Patent Office
Office européen des brevets

(11) Publication number: **0 270 088 B1**

(12) **EUROPEAN PATENT SPECIFICATION**

(45) Date of publication of patent specification: **02.12.92**

(51) Int. Cl.5: **A61B 5/00**, G01N 27/416

(21) Application number: **87117811.7**

(22) Date of filing: **02.12.87**

(54) **Automatic calibration apparatus for a partial gas pressure measuring sensor.**

(30) Priority: **05.12.86 JP 291356/86**
**05.12.86 JP 291357/86**

(43) Date of publication of application:
**08.06.88 Bulletin 88/23**

(45) Publication of the grant of the patent:
**02.12.92 Bulletin 92/49**

(84) Designated Contracting States:
**DE ES FR GB IT**

(56) References cited:
**EP-A- 0 074 498**
**DE-A- 2 608 727**
**GB-A- 1 470 155**

**PATENT ABSTRACTS OF JAPAN, vol. 6, no. 156 (P-135)[1034], 17th August 1982; & JP - A - 57 74651**

(73) Proprietor: **SUMITOMO ELECTRIC INDUSTRIES, LTD.**
**5-33, Kitahama 4-chome, Chuo-ku**
**Osaka-shi, Osaka 541(JP)**

(72) Inventor: **Miyagawa, Yuichi c/o Osaka Works**
**Sumimoto Electric Ind. Ltd. 1-3, Shimaya 1-chome**
**Konohana-ku Osaka(JP)**
Inventor: **Yoneda, Kiwamu c/o Osaka Works**
**Sumimoto Electric Ind. Ltd. 1-3, Shimaya 1-chome**
**Konohana-ku Osaka(JP)**
Inventor: **Kuwa, Kazuhiro c/o Osaka Works**
**Sumimoto Electric Ind. Ltd. 1-3, Shimaya 1-chome**
**Konohana-ku Osaka(JP)**
Inventor: **Kogo, Katsuyuki c/o Osaka Works**
**Sumimoto Electric Ind. Ltd. 1-3, Shimaya 1-chome**
**Konohana-ku Osaka(JP)**

(74) Representative: **Patentanwälte Kirschner & Grosse**
**Forstenrieder Allee 59**
**W-8000 München 71(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 270 088 B1**

## Description

The present invention relates to an automatic calibration apparatus for a partial gas pressure measuring sensor. More particularly, the present invention relates to an automatic calibration apparatus for a partial gas pressure measuring sensor, in which an output of a transcutaneous partial oxygen pressure measuring sensor or the like is automatically calibrated in atmospheres of at least two kinds of standard gases.

Description of the Prior Art

It is a widely utilized method to measure a partial pressure of carbon dioxide gas, oxygen gas or the like in blood by means of an electrochemical sensor such as a transcutaneous oxygen pressure measuring sensor. If such an electrochemical sensor is used to measure partial pressure of gas, the measurement is effected while calibration of the sensor is being done. In the following, an example of measurement of partial pressure of carbon dioxide (referred to hereinafter as $PCO_2$) will be described. Since a $PCO_2$ sensor shows a fixed potential value with respect to an identical value of $PCO_2$, automatic calibration is performed by a two-point calibration method using known $PCO_2$, gases as standard gases, i.e., generally two kinds of air-mixed gases having different carbon dioxide partial gas pressures (those two gases having a lower and a higher partial gas pressure being referred to hereinafter as "low gas" and "high gas", respectively).

In a conventional automatic calibration apparatus for the $PCO_2$ sensor, it is necessary to avoid calibration in a state in which the low gas or the high gas is not normally supplied. For this reason, calibration using the low gas (hereinafter referred to as "low calibration") and calibration using the high gas (hereinafter referred to as "high calibration") are performed and comparison is made between an output potential of the $PCO_2$ sensor at the time of low calibration and an output potential of the $PCO_2$ sensor at the time of high calibration. If the difference between those potentials is within a predetermined range, it is determined that the high gas and the low gas are both supplied normally and that the calibrations are normally performed.

On the other hand, if the potential difference is outside the predetermined range, it is determined that the high gas and the low gas are not normally supplied and that the calibrations are not normally performed, whereby the calibrations are newly performed. Thus, if the high gas and the low gas are not normally supplied at the time of automatic calibration operation, it is necessary to newly perform the low calibration and the high calibration, which takes much time in the automatic calibration operation.

From the DE-A-2 608 727, a partial gas pressure measuring method and apparatus is known. For measuring the partial pressure of oxygen, a polarographic gas measurement electrode is used. The gas supply to the electrode is switched periodically to a reference gas in order to carry out an automatic calibration of the apparatus after certain periods of time.

Therefore, a primary object of the present invention is to provide an automatic calibration apparatus for a partial gas pressure measuring sensor, in which calibration is immediately stopped when it is determined that one of at least two kinds of standard gases is abnormally supplied, whereby a period of automatic calibration can be shortened.

In an automatic calibration apparatus for a partial gas pressure measuring sensor according to another aspect of the present invention, the partial gas pressure measuring sensor is intermittently exposed to a standard gas atmosphere, which makes it possible to improve a measuring precision after the exposure and to reduce gas consumption.

Briefly stated, at least two kinds of standard gases are individually supplied to gas selection means from standard gas suppliers through gas supply paths and any of the at least two kinds of standard gases are selected by the gas selection means so as to be supplied to a sensor placing means. When the partial gas pressure measuring sensor is placed on the sensor placing means, the sensor is exposed to an atmosphere of the standard gas and an output potential value of the partial gas pressure measuring sensor is stored in storing means. The gas supply paths are provided with detection means for detecting abnormal supply of the standard gases and when the detection means detects abnormal supply of any standard gas, operation for storing the output potential value of the sensor in the storing means is stopped.

Thus, according to the present invention, when abnormal supply of any of the standard gases is detected, storage of the output potential value of the partial gas pressure measuring sensor in the storing means is immediately stopped and consequently there is no fear of terminating a sequence of processing for automatic calibration without effecting normal calibration.

In a preferred embodiment of the present invention, there is provided notification means for notifying abnormal supply of standard gas when such abnormal supply of the standard gas is detected.

In a further preferred embodiment of the present invention, procedures for performing automatic calibration are selected and based on the selection, at least two kinds of standard gases are selected, so that automatic calibration is performed corresponding to the selected standard gas.

In a further preferred embodiment of the present invention, removal of the partial gas pressure measuring sensor from the automatic calibration apparatus is detected and procedures for performing automatic calibration are changed based on an output of the detection.

In a still further preferred embodiment of the present invention, when the partial gas pressure measuring sensor is placed on the sensor placing means, a period of supply of a standard gas starts to be measured and when the measured period attains a prescribed time, the supply is stopped. Thus, the standard gas is intermittently supplied by the gas selection means to the sensor placing means, so that the partial gas pressure measuring sensor is held in the atmosphere of a predetermined potential gas pressure.

These objects and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a schematic block diagram for explaining a fundamental feature of the present invention.

Fig. 2 is a concrete block diagram of a first embodiment of the present invention.

Fig. 3 is an illustration showing gas supply paths for standard gases.

Fig. 4 is an illustration showing a structure of a sensor spot.

Fig. 5 is a flow chart showing a main routine of the above stated embodiment.

Fig. 6 is a flow chart showing a subroutine for stopping automatic calibration.

Fig. 7 is a flow chart showing a subroutine for waiting.

Fig. 8 is a flow chart showing a clock signal interruption processing routine.

Figs. 9A and 9B are diagrams showing a connection portion of a $PCO_2$ sensor in a second embodiment of the present invention.

Fig. 10 is a flow chart showing a clock signal interruption processing routine in the second embodiment.

Fig. 11 is a flow chart showing a main routine of the second embodiment.

Fig. 12 is a block diagram for explaining an outline of a third embodiment of the present invention.

Fig. 13 is flow chart showing a subroutine for stopping automatic calibration in the third embodiment.

Fig. 14 is a flow chart showing a subroutine for waiting in the third embodiment.

Fig. 15 is a flow chart showing a clock signal interruption processing routine in the third embodiment.

Figs. 16 to 18 are graphs showing changes in potential of a $PCO_2$ sensor in the third embodiment.

DESCRIPTION OF THE PREFERRED EMBODIMENTS

Fig. 1 is a diagram for explaining an outline of an operation principle of the present invention. A gas supplier 101 supplies the high gas and the low gas. The high gas and the low gas from the gas supplier 101 are supplied to gas selection means 103 through abnormal gas supply detection means 102. The abnormal gas supply detection means 102 detects abnormal supplies of the low gas and the high gas. The gas selection means 103 selectively supplies the high gas and the low gas to a sensor placing portion 104. The sensor placing portion 104 places a partial gas pressure sensor 105 thereon and the partial gas pressure sensor 105 thus placed is exposed to an atmosphere of the high gas or the low gas selected by the gas selection means 103, so that automatic calibration is performed. More specifically, an output potential value of the partial gas pressure sensor 105 is stored in storing means 107 by means of automatic calibration control means 106. When abnormal supply of the high gas or the low gas is detected by the abnormal gas supply detection means 102, the automatic calibration control means 106 stops storage of the output potential value of the sensor 105 in the storing means 107.

Fig. 2 is a schematic block diagram of a first embodiment of the present invention; Fig. 3 is an illustration showing gas supply paths in this embodiment; and Fig. 4 is a illustration showing a structure in a sensor spot.

Referring to Figs. 2 to 4, a construction of this embodiment will be described. When a $PCO_2$ sensor 5 is placed on a spot 27, it is exposed to an atmosphere of standard gas and an output potential of the sensor 5 is supplied to an A/D converter 6, where it is converted to a digital signal. The digital signal is supplied to a central processing unit (CPU) 12 through an input port 7. The input port 7 is connected with a pressure sensitive switch 8 for detection of abnormal supply of the low gas, a pressure sensitive switch 9 for detection of abnormal supply of the high gas, an automatic calibration switch 10, and an automatic calibration procedure selection switch 11. Contacts of those switches 8 to 11 on one side are connected to a power supply Vcc and contacts thereof on the other side are grounded.

The pressure sensitive switch 8 for detection of abnormal supply of the low gas and the pressure sensitive switch 9 for detection of abnormal supply of the high gas are connected to gas supply paths shown in Fig. 3 to be described afterwards. If those switches 8 and 9 are set to the respective contacts on the grounding side, this means that the low gas and the high gas are normally supplied. If abnormal supply of the low gas occurs and a pressure in the gas supply circuit becomes lower than a predetermined value, the pressure sensitive switch 8 for detection of abnormal supply of the low gas is turned to the contact on the side of the power supply Vcc. Similarly, if abnormal supply of the high gas occurs, the pressure sensitive switch 9 for detection of abnormal supply of the high gas is turned to the contact on the side of the power supply Vcc. The automatic calibration switch 10 is used to select execution of automatic calibration and the automatic calibration procedure selection switch 11 is used to select execution of low calibration or high calibration.

The CPU 12 is connected with the above stated input port 7 and is also connected with a clock generator 13, an ROM 14, an RAM 15 and an output port 16. A program for controlling the CPU 12 is stored in advance in the ROM 14. The CPU 12 performs various operations based on this program. For example, it receives necessary data from the input port 7, communicates data with the RAM 15, determines a period by counting based on a clock signal supplied from the clock generator 13, or outputs necessary data to the output port 16. The RAM 15 is used to store various data. It includes an area 15 as a counter for determining time.

The output port 16 is connected with a display portion 17 and two-directional electromagnetic valves 18 and 19. The display portion 17 displays a message indicating abnormal supply of the low gas or the high gas dependent on a signal supplied from the output port 16. The two-directional electromagnetic valves 18 and 19 select supply of the high gas or the low gas dependent on an output of the output port 16.

Referring to Fig. 3, a construction of the gas supply paths will be described. A low gas cylinder 21 supplies the low gas and a high gas cylinder 22 supplies the high gas. The low cylinder 21 and the high gas cylinder 22 are connected to pressure regulators 23 and 24, respectively, through pipes. Those pressure regulator 23 and 24 regulate pressures of the low gas and the high gas, respectively, to about 1.5 kg/cm$^2$ for example. Output portions of the pressure regulators 23 and 24 are connected with the pressure sensitive switch 8 for detection of abnormal supply of the low gas and the pressure sensitive switch 9 for detection of abnormal supply of the high gas, respectively. Output portions of those switches 8 and 9 are connected to flow rate regulators 25 and 26, respectively, through pipes. Those flow rate regulators 25 and 26 regulate flow rates of the low gas and the high gas, respectively, to about 5 ml/min. for example. In addition, output portions of the flow rate regulators 25 and 26 are connected to the above stated two-directional electromagnetic valves 18 and 19 shown in Fig. 2, respectively. Output portions of the two-directional electromagnetic valves 18 and 19 are connected to a sensor spot 27.

The $PCO_2$ sensor 5 is placed on the sensor spot 27, where the $PCO_2$ sensor 5 is calibrated. For this purpose, the sensor spot 27 is provided as shown in Fig. 4. More specifically, the low gas or the high gas introduced through the gas supply paths described above referring to Fig 3 is introduced from the gas inlet path 273 to a gas reservoir 272, so that a sensitive surface 51 of the $PCO_2$ sensor 5 is exposed to a atmosphere of the low gas or an atmosphere of the high gas. A gas outlet path 274 is provided to maintain pressure in the gas reservoir 272 to be substantially equal to an atmospheric pressure and to rapidly replace the gas in the gas reservoir 272 by another gas. The $PCO_2$ sensor 5 is pressed against a ring-shaped packing 271 by means of a rotating arm 275, so that the gas can be prevented from leaking out between the $PCO_2$ sensor 5 and the sensor spot 27.

Fig. 5 is a flow chart showing a main routine of this embodiment; Fig. 6 is a flow chart showing a subroutine for stopping automatic calibration; Fig. 7 is a flow chart showing a subroutine for waiting; and Fig. 8 is showing a clock signal interruption processing routine.

Referring to Figs. 2 to 8, a concrete operation of this embodiment will be described. First, the $PCO_2$ sensor 5 is placed on the sensor spot 27 shown in Fig. 4 and execution of automatic calibration is selected by operating the automatic calibration switch 10. Then, a program of the main routine shown in Fig. 5 is started. First in the step SP1, the CPU 12 clears a time counter 151 for measuring time. The time counter 151 is incremented in the interruption processing routine of Fig. 8 executed in response to a clock signal inputted from the clock generator 13 to the CPU 12 with a predetermined cycle. In the step SP2, the CPU 12 opens the two-directional electromagnetic valve 18 for the low gas and closes the two-directional electromagnetic valve 19 for the high gas. As a result, the pressure of the low gas supplied from the low gas cylinder 21 is regulated by the pressure regulator 23 and the flow rate thereof is regulated by the flow rate regulator 25, so that the low gas is supplied to the sensor spot 27.

The CPU 12 determines in the step SP3 whether the low gas is normally supplied or not. This determination is effected by reading an output voltage of the pressure sensitive switch 8 for detection of abnormal supply of the low gas through the input port 7. More specifically, if the output voltage of this pressure sensitive switch 8 is 0V, it is determined that the low gas is normally supplied. If the output voltage is Vcc, it is determined that the low gas is not normally supplied. If the low gas is not normally supplied, the CPU 12 proceeds to the step SP4 to execute processing for stopping automatic calibration shown in Fig. 6. Thus, the CPU 12 displays a message in the display portion 12 indicating that abnormal supply of the low gas occurs.

On the other hand, if the low gas is normally supplied, the CPU 12 proceeds to the step SP5 to determine based on an output of the time counter 151 whether time T1 has passed or not. If the time T1 has not passed, the CPU 12 repeats the operations in the steps SP2 and SP3 until the output of the time counter 151 indicates a lapse of the time T1. This repetition is required for waiting until the $PCO_2$ sensor 5 placed on the sensor spot 27 is kept in a stable state in the atmosphere of the low gas. If abnormal supply of the low gas occurs before the time T1 has passed, the operation for stopping automatic calibration in the step SP4 is executed.

If it it determined in the step SP5 based on the output of the time counter 151 that the time T1 has passed, the CPU 12 reads an output potential PL of the $PCO_2$ sensor converted by the A/D converter 6 through the input port 7 and stores it in the RAM 15. The CPU 12 determines in the step SP7 whether execution procedures are selected by the automatic calibration procedure selection switch 11. If the execution procedures are not selected by the automatic calibration procedure selection switch 11, the CPU 12 proceeds to the step SP8 to execute the routine for waiting shown in Fig. 7.

More specifically, the CPU 12 closes the two-directional electromagnetic valves 18 and 19 in the step SP81 to stop supply of the gas and clears the time counter 151 in the step SP82. Then, in the step SP83, the CPU 12 determines whether the automatic calibration switch 10 is turned on or not. If it is not turned on, automatic calibration is brought to an end in the step SP84. If the automatic calibration switch 10 is turned on, it is determined in the step SP85 whether the output of the time counter 151 indicates a lapse of time T3 or not. If the time T3 has not passed, the operations in the steps SP83 and SP85 are repeated. If it is determined that the time T3 has passed, the CPU 12 returns again to the main routine shown in Fig. 5.

If it is determined in the step SP7 that the execution procedures are selected by the automatic calibration procedure selection switch 11, the CPU 12 clears the time counter 151 in the step SP9. Then, in the step SP10, the CPU 12 closes the two-directional electromagnetic valve 18 for the low gas and opens the two-directional electromagnetic valve 19 for the high gas, so as to supply the high gas to the sensor spot 27. Thus, a pressure of the high gas from the high gas cylinder 22 is regulated by the pressure regulator 24 and a flow rate thereof is regulated by the flow rate regulator 26. In such a manner, the high gas is supplied to the sensor spot 27 through the two-directional electromagnetic valve 19. The CPU 12 determines in the step SP11 whether the high gas is normally supplied or not.

If the high gas is not normally supplied, the CPU 12 stops automatic calibration in the step SP12 and displays a message in the display portion 17 indicating abnormal supply of the high gas. If the high gas is normally supplied, the CPU 12 determines in the step SP13 whether time T2 has passed or not, based on an output of the time counter 151. If the time T2 has not passed, the operations in the steps SP10 and SP11 are repeated. If the time T2 has passed, the CPU 12 reads an output potential PH of the $PCO_2$ sensor 5 converted as a digital signal by the A/D converter 6 through the input port 7 and stores the output potential PH in the RAM8. Then, the CPU 12 executes the processing routine for waiting shown in Fig. 7.

Now, procedures to be executed on the occurrence of abnormal supply of the low gas or the high gas will be described. When abnormal supply of the low gas or the high gas is detected, the message indicating abnormal supply of the gas is displayed in the display portion 17 and the automatic calibration program is brought to an end. Then, the user executes procedures for recovery such as replacement of the low gas cylinder 21 or the high gas cylinder 22 and selects again execution of automatic calibration by using the automatic calibration switch 10 to restart the automatic calibration program shown in Fig. 5.

Figs. 9A and 9B are diagrams showing connection between the $PCO_2$ sensor and the automatic calibration apparatus in a second embodiment of the present invention. Particularly, Fig. 9A shows a state in which the $PCO_2$ sensor is not connected and Fig. 9B shows a state in which the $PCO_2$ sensor is connected.

In Figs. 9A and 9B, a connector 31 is provided in the $PCO_2$ sensor and terminals 32 and 33 are short-circuited. A connector 34 is provided in the automatic calibration apparatus and a terminal 35 is connected to the input port 7 as well as to a DC power supply Vcc through a pull-up resistor 37 with a terminal 36 being grounded. Accordingly, if the $PCO_2$ sensor 5 shown in Fig. 2 is not connected to the apparatus, a voltage Vcc is applied to the input port 4. On the other hand, if the $PCO_2$ sensor 5 is connected to the apparatus, a voltage 0V is applied to the input port 4 since the terminals 32 and 33 of the connector 31 in the $PCO_2$ sensor 5 are connected to he terminals 35 and 36 of the connector 34 in the apparatus, respectively.

Fig. 10 shows a clock signal interruption processing routine in the second embodiment, and Fig. 11 shows a main routine in this embodiment.

Concrete operation of this embodiment will be described in the following. When a clock signal is supplied from the clock generator 13 to the CPU 12, a program executed at that time is temporarily suspended, and the CPU 12 proceeds to the clock signal interruption processing routine shown in Fig. 10. Then, in the step SP21, the CPU 12 increments the time counter 151 by 1 and determines in the step SP22 whether the $PCO_2$ sensor 5 is connected to the automatic calibration apparatus or not. This determination is effected by reading a voltage inputted from the connector 34 to the input port 7. More specifically, if the voltage inputted to the input port 7 is the power supply voltage Vcc, the CPU 12 determines that the $PCO_2$ sensor 5 is not connected. If it is 0V, the CPU 12 determines that the $PCO_2$ sensor 5 is connected.

If the $PCO_2$ sensor 5 is not connected, a sensor flag stored in the RAM 15 is reset to 0 in the step SP23, so that the CPU 12 proceeds to the step SP26 to execute the routine for waiting shown in Fig. 7. On the other hand, if it is determined that the $PCO_2$ sensor 5 is connected, the CPU 12 determines in the step SP24 whether the sensor flag is 0 or not. If the sensor flag is 0, a value of the sensor flag is set to 2, so that execution of the clock signal interruption processing routine is brought to an end to restart execution of the suspended program. The value of the sensor flag is a variable indicating a state of connection of the $PCO_2$ sensor 5. The flag "0" represents a state in which the $PCO_2$ is not connected, while the flags "1" and "2" both represent a state in which the $PCO_2$ sensor 5 is connected. Particularly, the flag "2" represents a state in which the $PCO_2$ sensor 5 not connected changes its state to be connected, but automatic calibration is not executed yet. The flag "1" represents the other states.

Referring to Fig. 11, the main routine will be described. The steps SP1 to SP6 in Fig. 11 are the same as in Fig. 5 and therefore description thereof is omitted. The CPU 12 determines in the step SP16 whether the sensor flag is 2 or not. If the sensor flag is not 2, the CPU 12 proceed to the step SP8 to execute the subroutine for waiting. On the other hand, if the sensor flag is 2, the sensor flag is set to 1 in the step SP17 and then in the same manner as described above in connection with Fig. 5, operations in the steps SP9 to SP15 are executed. More specifically, in this second embodiment, when the CPU 12 determines that the $PCO_2$ sensor 5 is replaced by a new one, which is newly connected, it automatically selects execution procedures for automatic calibration.

As described above, in this second embodiment, the two-point calibration operation including the low calibration and the high calibration is executed only when both the low calibration and the high calibration are required, for example, in case in which the $PCO_2$ sensor 5 connected is replaced by a new one. In other cases, it becomes possible to perform only the one-point calibration, i.e., either the low calibration or the high calibration. As a result, a period of time required for executing automatic calibration can be shortened.

Although the invention is applied to the automatic calibration apparatus for the $PCO_2$ sensor 5 in the above described respective embodiments, the present invention is not limited thereto and, needless to say, it is applicable to transcutaneous oxygen partial pressure sensor, a combined sensor which transcutaneously measures oxygen partial pressure and carbon dioxide partial pressure, or the like.

Thus, according to the above described respective embodiments of the present invention, while any of two or more kinds of standard gases is supplied to the sensor spot where the partial pressure measuring sensor is exposed to a standard gas atmosphere, automatic calibration is stopped upon detection of abnormal supply of the standard gas. Consequently, it becomes possible to eliminate the disadvantage of the conventional apparatus that abnormal supply of the standard gas cannot be detected without executing automatic calibration by the two-point calibration method using two kinds of standard gases. Thus, the period of time required for execution of automatic calibration can be reduced.

Fig. 12 is a diagram for explaining an outline of a third embodiment of the present invention. In this third embodiment, as shown in Fig. 12, gas supply time measuring means 109 measures time for supplying the high gas or the low gas, and sensor setting input means 110 inputs a signal indicating that the above stated $PCO_2$ sensor 105 is placed on the sensor placing portion 104. Gas selection means 112 is driven by gas supply state control means 111, whereby the $PCO_2$ sensor 105 is intermittently exposed to an atmosphere of the low gas or the high gas if the $PCO_2$ sensor 105 is placed on the sensor spot 104 but calibration is not performed.

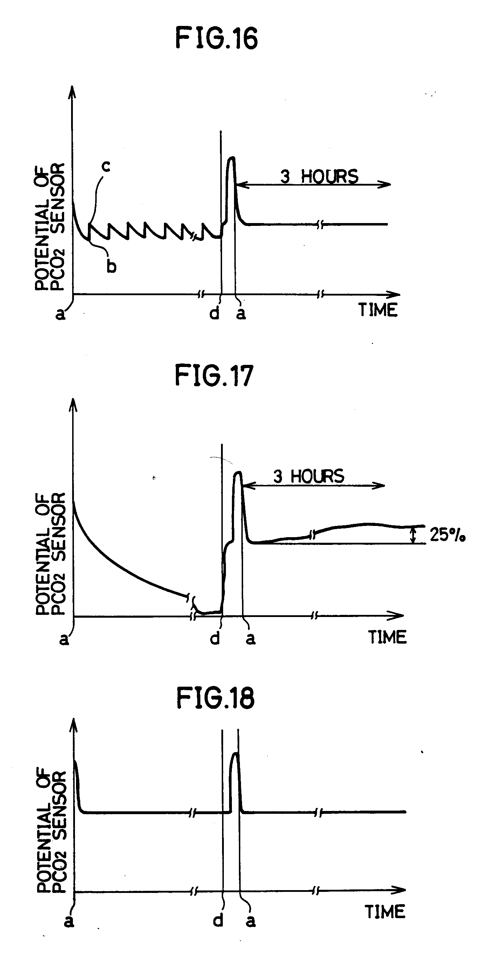

Fig. 13 is a flow chart showing an automatic calibration stop routine in the third embodiment; Fig. 14 is a flow chart showing a waiting routine in this third embodiment; and Fig. 15 is a flow chart showing a clock signal interruption processing routine in this embodiment.

The automatic calibration apparatus in this embodiment is constructed in the same manner as shown in Figs. 2 to 4. The automatic calibration routine of this embodiment is the same as described above in connection with Fig. 5. Therefore, detailed description thereof is omitted. First, when the power supply is turned on, the automatic calibration stop program shown in Fig. 13 is started. More specifically, the CPU 12 supplies signals to the two-directional electromagnetic valves 18 and 19 through the output port 16 in the step SP101, thereby to close those two-directional electromagnetic valves 18 and 19. Subsequently, in the step SP102, the CPU 12 reads a state of the sensor setting switch 10 through the input port 7 and determines whether the sensor setting on the sensor spot is selected or not. This determination is repeated until the user sets the $PCO_2$ sensor 5 on the sensor spot 27 and selects execution of automatic calibration by using the sensor setting switch 10.

If the sensor setting on the sensor spot is selected, the CPU 12 determines in the step SP103 whether the low gas is normally supplied or not and determines in the step SP105 whether the high gas is normally supplied or not. Those determinations are effected by reading states of the pressure sensitive switch 8 for detection of abnormal supply of the low gas and the pressure sensitive switch 9 for detection of abnormal supply of the high gas through the input port 4. More specifically, if the output voltages of the pressure sensitive switch 8 for detection of abnormal supply of the low gas and the pressure sensitive switch 9 for detection of abnormal supply of the high gas are identical to the potential of the power supply Vcc, it is determined that abnormal supply occurs. If each of the output voltages are 0V, it is determined that each supply is normal.

If the CPU 12 determines that the low gas is not normally supplied, it displays, in the step SP104, a message in the display portion 17 representing abnormal supply of the low gas. If the CPU 12 determines that the high gas is not normally supplied, it displays, in the step SP106, a message in the display portion 17 representing abnormal supply of the high gas. On the other hand, if it is determined in the steps SP103 and SP105 that the low gas and the high gas are both normally supplied, it executes, in the step S107, automatic calibration as described above in connection with Fig. 5.

Referring now to Fig. 14, an example of the waiting routine in this embodiment will be described. In waiting operation, the CPU 12 clears the time counter 1 in the step SP31 and clears the time counter 2 in the step SP32. The value of the time counter 2 is a variable having the same function as that of the time counter 1 and is stored in the RAM 15 by a program. Then, the CPU 12 opens the two-directional electromagnetic valve 18 in the step SP33 to start supply of the low gas and determines in the step SP34 whether the low gas is normally supplied or not. Then, in the step SP36, it is determined based on the value of the time counter 2 whether the time T3 has passed after the start of supply of the low gas. If the time T3 has not passed, the operations in the steps of SP34 and SP36 are repeated. If abnormal supply of the low gas occurs before the lapse of the time T3, the automatic calibration stop program shown in Fig. 13 is executed. On the other hand, if it is determined in the step SP36 that the time T3 has passed, the CPU 12 clears the time counter 2 in the step SP37 and closes the two-dimensional electromagnetic valve 18 in the step SP38 to stop supply of the low gas.

Further in the step SP39, the CPU 12 determines whether the time counter 2 has measured time T4 or not and if not, it waits for a lapse of the time T4. When the CPU 12 determines in the step SP39 that the time counter 2 has measured the time T4, it determines in the step SP40 whether the time counter 1 has measured time T5 or not. If the time T5 has not passed, the CPU 12 returns to the above stated step SP32 and repeats the operations in the steps SP32 to SP40 until the time T5 has passed. When it is determined based on the value of the time counter 1 that the time T5 has passed, the CPU 12 executes the automatic calibration program in the step SP41.

Referring now to Fig. 15, an example of the clock signal interruption processing routine in this embodiment will be described. In this example, when a clock signal is inputted from the clock generator 13 to the CPU 12, a program executed at that time is temporarily suspended and the clock signal interruption processing routine is executed. When the execution of this routine is completed, the suspended program restarts to be executed. The CPU 12 increments by 1 in the steps SP51 and S52 the variable values of the time counter 1 and the time counter 2 stored in the RAM 15, respectively. Then in the step SP53, the CPU 12 determines whether the sensor setting switch 10 is turned on or not. If the sensor setting switch 10 is turned on, the interruption processing is brought to an end to restart execution of the suspended program. On the other hand, if the sensor switch 5 is turned off, the CPU 12 proceeds to the step SP54 without restarting execution of the suspended program, so as to execute the automatic calibration stop program shown in Fig. 13.

Figs. 16 to 18 are graphs showing potential changes of the $PCO_2$ sensor in the third embodiment.

In Fig. 16, the point a represents the time of end of automatic calibration operation, and after that, waiting operation starts. The point b represents the time of start of supply of the low gas in a waiting state; the point c represents the time of stop of the supply of the low gas; and the point d represents the time of start of the second automatic calibration operation. After the second automatic calibration operation is terminated, the $PCO_2$ sensor 5 is exposed to the atmosphere of the low gas so that a measuring experiment is conducted.

Fig. 17 shows a potential change of the $PCO_2$ sensor 5 in the case in which the low gas is not supplied in the waiting state after the end of the first automatic calibration. In the same manner as in the case of Fig. 16, the $PCO_2$ sensor 5 is exposed to the atmosphere of the low gas after the end of the second automatic calibration.

Fig. 18 shows a potential change of the $PCO_2$ sensor 5 in the case in which the low gas is continuously supplied in the waiting state after the end of the first automatic calibration. In this case also, the $PCO_2$ sensor 5 is exposed to the atmosphere of the low gas after the end of the second automatic calibration in the same manner as in the cases of Figs. 16 and 17.

Comparing the case of Fig. 16 with the case of Fig. 17, the time required for establishing a stable state of the $PCO_2$ sensor 5 in the second calibration is shorter in the case of Fig. 16 than that in the case of Fig. 17. In addition, as to the result of the measuring experiment conducted after the end of the second calibration, in the case of Fig. 17, there was an initial drift of 25 % for three hours after the start of the measurement of the experiment, while there was little drift in the case of Fig. 16 as well as in the case of Fig. 18. Furthermore, in the case of Fig. 16, a ratio between the low gas supply time and the gas supply stop time is in a range from 1 : 30 to 1 : 40 and the low gas consumption in the waiting state is 1/30 to 1/40 of that in the case of Fig. 18.

Although the invention is applied to the $PCO_2$ sensor also in the above described third embodiment, the present invention is not limited thereto and, needless to say, it is applicable to a transcutaneous oxygen partial pressure sensor, a combined sensor which transcutaneously measures oxygen partial pressure and carbon dioxide partial pressure, or the like.

Thus, according to the third embodiment of the present invention, the partial gas pressure measuring sensor placed on the sensor spot is intermittently exposed to the atmosphere of the standard gas at predetermined time intervals. Consequently, the measurement precision after that can be improved and the gas consumption can be reduced.

**Claims**

1. An automatic calibration apparatus for a partial gas pressure measuring sensor (105), for calibrating said sensor in atmospheres of at least two kinds of standard gases, comprising

    standard gas suppliers (21 and 22) for supplying said at least two kinds of standard gases,

    sensor placing means (27) on which said partial gas pressure measuring sensor to be calibrated is placed, for exposing said sensor to the atmospheres of said standard gases,

    a gas supply path (20) for individually introducing said at least two kinds of standard gases from said standard gas suppliers to said sensor placing means,

    detection means (8 and 9) coupled to said gas supply path for detecting abnormal supply of said standard gases,

    gas selection means (18 and 19) connected to said gas supply path for selecting any of said at least two kinds of standard gases to be supplied from said standard gas suppliers and supplying the selected gas to said sensor placing means,

    storing means (15) for storing an output of said partial gas pressure measuring sensor, and

    control means (12) for storing the output of said partial gas pressure measuring sensor in said storing means when the partial gas measuring sensor placed on said sensor placing means is exposed to the atmospheres of the standard gases, and stopping the storage of the output of said partial gas pressure measuring sensor in said storing means when said detection means detects abnormal supply of the standard gases.

2. An automatic calibration apparatus for a partial gas pressure measuring sensor in accordance with claim 1, further comprising

    procedure selection instructing means (11) for issuing an instruction for selecting procedures of execution of automatic calibration of said sensor,

    said control means comprising automatic calibration executing means responsive to the instruction from said procedure selection instructing means for enabling said gas selection means to select any of said at least two kinds of standard gases and executing automatic calibration corresponding to the selected standard gas.

**3.** An automatic calibration apparatus for a partial gas pressure measuring sensor in accordance with claim 2, further comprising

connection means (31 and 32) for connecting said partial gas pressure measuring sensor to said control means in a removable manner,

said control means comprising means for changing procedures of execution of automatic calibration in response to determination that said partial gas pressure measuring sensor is removed from said control means by said connection means.

**4.** An automatic calibration apparatus for a partial gas pressure measuring sensor in accordance with claim 1, further comprising

automatic calibration instructing means (10) for issuing an instruction to execute automatic calibration of said sensor,

said control means comprising means for storing the output of said partial gas pressure measuring sensor in said storing means in response to the instruction from said automatic calibration instructing means.

**5.** An automatic calibration apparatus for a partial gas pressure measuring sensor in accordance with claim 1, further comprising

time measuring means (151) for measuring a period of supply of the standard gas selected by said gas selection means,

said control means comprising means for switching said selection means to supply any of said at least two kinds of standard gases to said sensor placing means each time said time measuring means has measured a predetermined period.

**6.** An automatic calibration apparatus for a partial gas pressure measuring sensor in accordance with claim 1, further comprising

means for issuing an alarm when said detection means detects abnormal supply of any of the standard gases.

**7.** An automatic calibration apparatus for a partial gas pressure measuring sensor in accordance with claim 1, wherein

said standard gas suppliers comprise gas cylinders (21 and 22) provided corresponding to the respective ones of said at least two kinds of standard gases,

said gas selection means comprises valve members (18 and 19) provided corresponding to the respective gas cylinders, and

said gas supply path comprises means for connecting said gas cylinders to said valve members and connecting output portions of said valve members to said sensor placing means.

**8.** An automatic calibration apparatus for a partial gas pressure measuring sensor in accordance with claim 7, wherein

said detection means comprise pressure detecting means (8 and 9) connected between said gas cylinders and said valve members for detecting pressures of said at least two kinds of standard gases.

**9.** An automatic calibration apparatus for a partial gas pressure measuring sensor in accordance with claim 7, further comprising

pressure regulating means (23 and 24) connected between said gas cylinders and said valve members for regulating pressures of said at least two kinds of standard gases.

**10.** An automatic calibration apparatus for a partial gas pressure measuring sensor in accordance with claim 7, further comprising

flow rate regulating means (25 and 26) connected between said gas cylinders and said valve members for regulating flow rates of said at least two kinds of standard gases.

**11.** An automatic calibration apparatus for a partial gas pressure measuring sensor in accordance with claim 7, wherein

said gas cylinders comprise cylinders for supplying standard gases having different gas partial pressures.

**12.** An automatic calibration apparatus for a partial gas pressure measuring sensor in accordance with claim 1, wherein

said partial gas pressure measuring sensor comprises any of a sensor for transcutaneously measuring partial pressure of oxygen, a sensor for transcutaneously measuring partial pressure of carbon dioxide gas, and a combined sensor for transcutaneously measuring partial pressure of oxygen and partial pressure of carbon dioxide.

**13.** An automatic calibration apparatus for a partial gas pressure measuring sensor, according to claim 1 characterized by :

time measuring means (151) for measuring a period of supply of the standard gas selected by said gas selection means,

instructing means (10) for instructing automatic calibration of said partial gas pressure measuring sensor, and

control means (12) for determining any of the standard gases to be supplied each time said time measuring means has measured a predetermined period in response to the instruction of said instructing means, thereby to control said gas selection means to supply the determined standard gas.

**14.** An automatic calibration method for a partial gas pressure measuring sensor, for calibrating said sensor in atmospheres of at least two kinds of standard gases, comprising the steps of:

placing said partial gas pressure measuring sensor on sensor placing means,

supplying any one of said at least two kinds of standard gases to said sensor placing means to expose said sensor to the atmosphere of the supplied standard gas,

storing an output of said partial gas pressure measuring sensor placed in the atmosphere of said standard gas, and

detecting a state of supply of said standard gas, stopping the storage of the output of said partial gas pressure measuring sensor upon detection of abnormal supply of said standard gas and notifying the abnormal supply.

**15.** An automatic calibration method for a partial gas pressure measuring sensor according to claim 14, characterized by,

instructing a start of automatic calibration of said partial gas pressure measuring sensor, and

starting measurement of a period after the issuance of said instruction of the start of the automatic calibration and changing the standard gas to be supplied to said sensor placing means each time a predetermined time has been measured.

## Patentansprüche

**1.** Automatisches Eichgerät für einen Partialgasdruckmeßfühler (105) zum Eichen des Fühlers in Atmosphären aus wenigstens zwei Arten von Standardgasen, mit

- Standardgaszuführeinrichtungen (21 und 22) zum Zuführen der wenigstens zwei Arten von Standardgasen,
- Fühleraufnahmeinrichtungen (27), auf denen der zu eichende Partialgasdruckmeßfühler angeordnet wird, um den Fühler den Atmosphären der Standardgase auszusetzen,
- einem Gaszufuhrweg (20) zum individuellen Zuführen der wenigstens zwei Arten von Standardgasen von den Standardgaszuführungseinrichtungen zur Fühleraufnahmeeinrichtung,
- Detektoreinrichtungen (8 und 9), die mit dem Gaszufuhrweg verbunden sind, um eine nicht der Norm entsprechende Zufuhr von Standardgasen zu erfassen,
- Gasauswahleinrichtungen (18 und 19), die mit dem Gaszufuhrweg verbunden sind, um ein beliebiges der wenigstens zwei Arten von Standardgasen, die von den Standardgaszufuhreinrichtungen bereitgestellt werden, auszuwählen und der Fühleraufnahmeeinrichtung das ausgewählte Gas zuzuführen,
- Speichereinrichtungen (15) zum Speichern von wenigstens einem Ausgangswert des Partialgasdruckmeßfühlers, und
- Steuereinrichtungen (12) zum Speichern des Ausgangwerts des Partialgasdruckmeßfühlers in der Speichereinrichtung, wenn der auf der Fühleraufnahmeeinrichtung angeordnete Partialgasdruckmeßfühler den Atmosphären der Standardgase ausgesetzt wird, und zum Abbrechen des Speicherns des Ausgabewerts des Partialgasdruckmeßfühlers in der Speichereinrichtung, wenn die Detektoreinrichtung eine nicht der Norm entsprechende Standardgaszufuhr feststellt.

**2.** Automatisches Eichgerät für einen Partialgasdruckmeßfühler nach Anspruch 1, welches weiterhin umfaßt:

- eine Verfahrensauswahlbefehlseinrichtung (11) zum Abgeben eines Befehls zur Auswahl von Verfahren zur Ausführung des automatischen Eichens des Meßgeräts,
- wobei die Steuereinrichtung automatische Einrichtungen zur Durchführung des Eichens aufweist, die auf den Befehl der Verfahrensauswahlbefehlseinrichtung ansprechen, um die Gasauswahleinrichtung in die Lage zu versetzen, eine der wenigstens zwei Arten von Standardgasen auszuwählen und das automatische Eichen entsprechend dem gewählten Standardgas durchzuführen.

**3.** Automatisches Eichgerät für einen Partialgasdruckmeßfühler nach Anspruch 2, welches weiterhin aufweist:

- Verbindungseinrichtungen (31 und 32), um den Partialgasdruckmeßfühler mit der Steuereinrichtung lösbar zu verbinden,

- wobei die Steuereinrichtung Einrichtungen zum Ändern der Verfahren zur Ausführung des automatischen Eichens aufweist, die darauf ansprechen, daß bestimmt wird, daß der Partialgasdruckmeßfühler durch die Verbindungseinrichtung von der Steuereinrichtung entfernt wird.

**4.** Automatisches Eichgerät für einen Partialgasdruckmeßfühler nach Anspruch 1, welches weiterhin aufweist:
- eine automatische Eichbefehlseinrichtung (10) zum Abgeben eines Befehls zur Durchführung des automatischen Eichens des Fühlers,
- wobei die Steuereinrichtung Einrichtungen zum Speichern des Ausgabewerts des Partialgasdruckmeßfühlers in der Speichereinrichtung aufweist, die auf den Befehl der automatischen Eichbefehlseinrichtung ansprechen.

**5.** Automatisches Eichgerät für einen Partialgasdruckmeßfühler nach Anspruch 1, welches weiterhin aufweist:
- Zeitmeßeinrichtungen (151), um eine Zufuhrzeitspanne für die Standardgase durch die Gaszufuhreinrichtung zu messen,
- wobei die Steuereinrichtung Einrichtungen zum Umschalten der Auswahleinrichtung aufweist, um eines der wenigstens zwei Arten von Standardgasen zur Fühleraufnahmeeinrichtung immer dann zuzuführen, wenn die Zeitmeßeinrichtung eine bestimmte Zeitspanne gemessen hat.

**6.** Automatisches Eichgerät für einen Partialgasdruckmeßfühler nach Anspruch 1, welches weiterhin aufweist:
- eine Einrichtung zum Abgeben eines Alarms, wenn die Detektoreinrichtung eine nicht der Norm entsprechende Zufuhr eines der Standardgase feststellt.

**7.** Automatisches Eichgerät für einen Partialgasdruckmeßfühler nach Anspruch 1, wobei
- die Standardgaszufuhreinrichtungen Gaszylinder (21 und 22) aufweisen, die entsprechend bei den jeweiligen der wenigstens zwei Arten von Standardgasen vorgesehen sind,
- wobei die Gasauswahleinrichtung Ventilelemente (18 und 19) aufweist, die entsprechend bei den jeweiligen Gaszylinder vorgesehen sind, und
- wobei der Gaszufuhrweg Einrichtungen zum Verbinden der Gaszylinder mit den Ventilelementen und zum Verbinden der Ausgangsbereich der Ventilelemente mit den Fühleraufnahmeeinrichtungen aufweist.

**8.** Automatisches Eichgerät für einen Partialgasdruckmeßfühler nach Ansrpuch 7, wobei
- die Detektoreinrichtung Druckerfassungseinrichtungen (8 und 9) aufweist, die zwischen den Gaszylindern und den Ventilelementen angeschlossen sind, um jeweils den Druck der wenigstens zwei Arten von Standardgasen zu erfassen.

**9.** Automatisches Eichgerät für einen Partialgasdruckmeßfühler nach Anspruch 7, welches weiterhin aufweist:
- Drucksteuereinrichtungen (23 und 24), die zwischen den Gaszylindern und den Ventilelementen angeschlossen sind, um den Druck der wenigstens zwei Arten von Standardgasen zu regulieren.

**10.** Automatisches Eichgerät für einen Partialgasdruckmeßfühler nach Anspruch 7, welche weiterhin aufweist:
- Durchflußregelungseinrichtungen (25 und 26), die zwischen den Gaszylindern und den Ventilelementen angeschlossen sind, um den Durchfluß der wenigstens zwei Arten von Standardgasen zu regulieren.

**11.** Automatisches Eichgerät für einen Partialgasdruckmeßfühler nach Anspruch 7, wobei
- die Gaszylinder Zylinder zum Zuführen der Standardgase mit unterschiedlichen Partialgasdrücken aufweisen.

**12.** Automatisches Eichgerät für einen Partialgasdruckmeßfühler nach Anspruch 1, wobei
- das Partialgasdruckmeßgerät einen Fühler zum transkutanen Messen des Partialdrucks von Sauerstoff, einen Fühler zum transkutanen Messen des Partialdrucks von Kohlendioxidgas oder aber einen kombinierten Fühler zum transkutanen Messen des Partialdrucks von Sauerstoff und des Partialdrucks von Kohlendioxid aufweist.

**13.** Automatisches Eichgerät für einen Partialgasdruckmeßfühler nach Anspruch 1, gekennzeichnet durch:
- Zeitmeßeinrichtungen (151), um eine Periode für das Zuführen des von der Gasauswahleinrichtung ausgewählten Standardgases zu messen,

- Befehlseinrichtungen (10), um den Befehl zum automatischen Eichen des Partialgasdruckmeßfühlers abzugeben, und
- Steuereinrichtungen (12) zum Bestimmen, daß jeweils dann eines der Standgase zugeführt wird, wenn die Zeitmeßeinrichtung durch Ansprechen auf einen Befehl der Befehlseinrichtung eine vorgegebene Periode gemessen hat, um dadurch die Gasauswahleinrichtung so zu steuern, daß das bestimmte Standardgas zugeführt wird.

**14.** Automatisches Eichverfahren für einen Partialgasdruckmeßfühler, um den Fühler in Atmosphären von wenigstens zwei Arten von Standardgasen zu eichen, wobei das Verfahren folgende Schritte umfaßt:

- Plazieren des Partialgasdruckmeßfühlers auf der Fühleraufnahmeeinrichtung,
- Zuführen von wenigstens einem von zwei Arten von Standardgasen zu der Fühleraufnahmeeinrichtung, um den Fühler der Atmosphäre der Standardgase auszusetzen,
- Speichern eines Ausgabewertes des in der Atmosphäre des Standardgases angeordneten Partialdruckmeßgeräts, und
- Erfassen des Zufuhrzustands des Standardgases, Abbrechen des Speicherns des Ausgabewerts des Partialgasdruckmeßfühlers nach Erfassen der nicht standardmäßigen Zufuhr von Standardgas und nach Registrieren der nicht normalen Zufuhr.

**15.** Automatisches Eichverfahren für einen Partialgasdruckmeßfühler nach Anspruch 14, gekennzeichnet durch:

- Befehlen des Starts des automatischen Eichens des Partialgasdruckmeßfühlers, und
- Starten der Messung einer Periode nach dem Abgeben des Befehls zum Starten des automatischen Eichens und und Wechseln des der Fühleraufnahmeeinrichtung zuzuführenden Standardgases jedesmal, wenn eine bestimmte Zeit gemessen wurde.

## Revendications

**1.** Appareil d'étalonnage automatique pour un détecteur (105) de mesure de pression de gaz partielle, pour l'étalonnage dudit détecteur dans des atmosphères comprenant au moins deux types de gaz de référence, comportant

des dispositifs d'alimentation (21 et 22) en gaz de référence, pour l'alimentation desdits gaz de référence d'au moins deux types,

un moyen de pose (27) de détecteur sur lequel est placé ledit détecteur de mesure de pression de gaz partielle devant être étalonné, pour exposer ledit détecteur aux atmosphères desdits gaz de référence,

un circuit (20) d'alimentation en gaz pour introduire individuellement lesdits gaz de référence d'au moins deux types depuis lesdits dispositifs d'alimentation en gaz de référence dans lesdits moyens de pose de détecteur,

des moyens de détection (8 et 9) couplés audit circuit d'alimentation en gaz pour détecter une alimentation anormale desdits gaz de référence,

des moyens de sélection de gaz (18 et 19) connectés audit circuit d'alimentation en gaz pour sélectionner l'un quelconque desdits gaz de référence d'au moins deux types devant être alimenté depuis lesdits dispositifs d'alimentation en gaz de référence et alimenter en gaz sélectionné ledit moyen de pose du détecteur,

un moyen de stockage (15) pour stocker un signal de sortie provenant dudit détecteur de mesure de pression de gaz partielle, et

un moyen de commande (12) pour stocker le signal de sortie dudit détecteur de mesure de pression de gaz partielle dans ledit moyen de stockage lorsque le détecteur de mesure de pression de gaz partielle placé sur ledit moyen de pose de détecteur est exposé aux atmosphères des gaz de référence et pour interrompre le stockage du signal de sortie dudit détecteur de mesure de pression da gaz partielle dans ledit moyen de stockage lorsque ledit moyen de détection détecte une alimentation anormale de gaz de référence.

**2.** Appareil d'étalonnage automatique pour un détecteur de mesure de pression de gaz partielle selon la revendication 1, comportant en outre

un moyen (11) d'instruction de sélection de procédure pour émettre une instruction de sélection de procédures d'exécution d'étalonnage automatique dudit capteur,

ledit moyen de commande comportant un moyen d'exécution de l'étalonnage automatique sensible à l'instruction provenant dudit moyen d'instruction de sélection de procédure pour permettre audit moyen de sélection de gaz de sélectionner l'un quelconque desdits gaz de référence d'au moins deux types et d'exécuter l'étalonnage automatique correspondant au gaz de référence sélectionné.

**3.** Appareil d'étalonnage automatique pour un détecteur de mesure de pression de gaz partielle selon la revendication 2, comportant en outre

des moyens de connexion (31 et 32) pour connecter de manière amovible ledit détecteur de mesure de pression de gaz partielle audit moyen de commande, ledit moyen de commande comprenant un moyen pour changer les procédures d'exécution d'étalonnage automatique en réponse à la détermination du fait que ledit détecteur de mesure de pression de gaz partielle est retiré dudit moyen de commande par lesdits moyens de connexion.

**4.** Appareil d'étalonnage automatique pour un détecteur de mesure de pression de gaz partielle selon la revendication 1, comportant en outre

un moyen (10) d'instruction d'étalonnage automatique pour émettre une instruction d'exécution d'étalonnage automatique dudit détecteur,

ledit moyen de commande comprenant des moyens de stockage du signal de sortie dudit détecteur de mesure de pression de gaz partielle dans ledit moyen de stockage en réponse à l'instruction provenant dudit moyen d'instruction d'étalonnage automatique.

**5.** Appareil d'étalonnage automatique pour un détecteur de mesure de pression de gaz partielle selon la revendication 1, comportant en outre

un moyen de mesure du temps (151) pour mesurer une période d'alimentation en gaz de référence sélectionné par ledit moyen de sélection de gaz,

ledit moyen de commande comportant un moyen pour connecter ledit moyen de sélection pour alimenter en un quelconque des gaz de référence d'au moins deux types ledit moyen de pose de détecteur chaque fois que ledit moyen de mesure de temps a mesuré une période prédéterminée.

**6.** Appareil d'étalonnage automatique pour un détecteur de mesure de pression de gaz partielle selon la revendication 1, comportant en outre

un moyen pour émettre une alarme lorsque ledit moyen de détection détecte une alimentation anormale en l'un quelconque des gaz de référence.

**7.** Appareil d'étalonnage automatique pour un détecteur de mesure de pression de gaz partielle selon la revendication 1, dans lequel

lesdits dispositifs d'alimentation en gaz de référence comportent des cylindres de gaz (21 et 22) contenant respectivement un desdits gaz de référence d'au moins deux types,

ledit moyen de sélection de gaz comportant des éléments de soupapes (18 et 19) prévues pour correspondre aux cylindres respectifs de gaz et

ledit circuit d'alimentation en gaz comportant un moyen pour connecter lesdits cylindres de gaz auxdits éléments de soupapes et pour connecter des parties de sortie desdits éléments de soupapes audit moyen de pose de détecteur.

**8.** Appareil d'étalonnage automatique pour un détecteur de mesure de pression de gaz partielle selon la revendication 7, dans lequel

lesdits moyens de détection comportent des moyens de détection de pression (8 et 9) connectés entre lesdits cylindres de gaz et lesdits éléments de soupapes pour détecter des pressions desdits gaz de référence d'au moins deux types.

**9.** Appareil d'étalonnage automatique pour un détecteur de mesure de pression de gaz partielle selon la revendication 7, comportant en outre

des moyens de réglage de pression (23 et 24) connectés entre lesdits cylindres de gaz et lesdits éléments de soupapes pour régler les pressions desdits gaz de référence d'au moins deux types.

**10.** Appareil d'étalonnage automatique pour un détecteur de mesure de pression de gaz partielle selon la revendication 7, comportant en outre

des moyens de réglage de débit (25 et 26) connectés entre lesdits cylindres de gaz et lesdits éléments de soupapes pour régler les débits desdits gaz de référence d'au moins deux types.

**11.** Appareil d'étalonnage automatique pour un détecteur de mesure de pression de gaz partielle selon la revendication 7, dans lequel

lesdits cylindres de gaz comportent des cylindres d'alimentation en gaz de référence ayant des pressions partielles de gaz différentes.

**12.** Appareil d'étalonnage automatique pour un détecteur de mesure de pression de gaz partielle selon la revendication 1, dans lequel

ledit détecteur de mesure de pression de gaz partielle comporte un détecteur quelconque pour la mesure transcutanée de la pression partielle d'oxygène, un détecteur pour la mesure transcutanée de la pression partielle de bioxyde de carbone et un détecteur combiné pour la mesure transcutanée de la pression partielle d'oxygène et la pression partielle de bioxyde de carbone.

**13.** Appareil d'étalonnage automatique pour un détecteur de mesure de pression de gaz partielle selon la revendication 1, caractérisé par :

un moyen de mesure de temps (151) pour mesurer une période d'alimentation en un gaz de référence sélectionné par ledit moyen de sélection de gaz,

un moyen d'instruction (10) pour donner une instruction d'étalonnage automatique dudit détecteur de mesure de pression de gaz partielle, et

un moyen de commande (12) pour déterminer lequel de l'un quelconque des gaz de référence doit être fourni chaque fois que ledit moyen de mesure du temps a mesuré une période prédéterminée en réponse à l'instruction dudit moyen d'instruction contrôlant ainsi ledit moyen de sélection de gaz pour alimenter en gaz de référence détermine.

**14.** Procédé d'étalonnage automatique pour un détecteur de mesure de pression de gaz partielle, pour étalonner ledit détecteur dans des atmosphères comprenant au moins deux types de gaz de référence, comportant les étapes qui consistent :

à placer ledit détecteur de mesure de pression de gaz partielle sur un moyen de pose de détecteur,

à alimenter en un quelconque desdits gaz de référence d'ait moins deux types ledit moyen de pose de détecteur pour exposer ledit détecteur à l'atmosphère du gaz de référence fourni,

à stocker un signal de sortie dudit détecteur de mesure de pression de gaz partielle placé dans l'atmosphère dudit gaz de référence, et

à détecter un état d'alimentation dudit gaz de référence, à interrompre le stockage du signal de sortie dudit détecteur de mesure de pression de gaz partielle au moment de la détection d'une alimentation anormale dudit gaz de référence et à signaler l'alimentation anormale.

**15.** Procédé d'étalonnage automatique pour un détecteur de mesure de pression de gaz partielle selon la revendication 14, caractérisé par les étapes qui consistent :

à donner une instruction de démarrage d'étalonnage automatique dudit détecteur de mesure de pression de gaz partielle,

à commencer la mesure d'une période après l'émission de ladite instruction du démarrage de l'étalonnage automatique et à changer le gaz de référence devant alimenter ledit moyen de pose de détecteur, chaque fois qu'un temps prédéterminé a été mesuré.

FIG.1

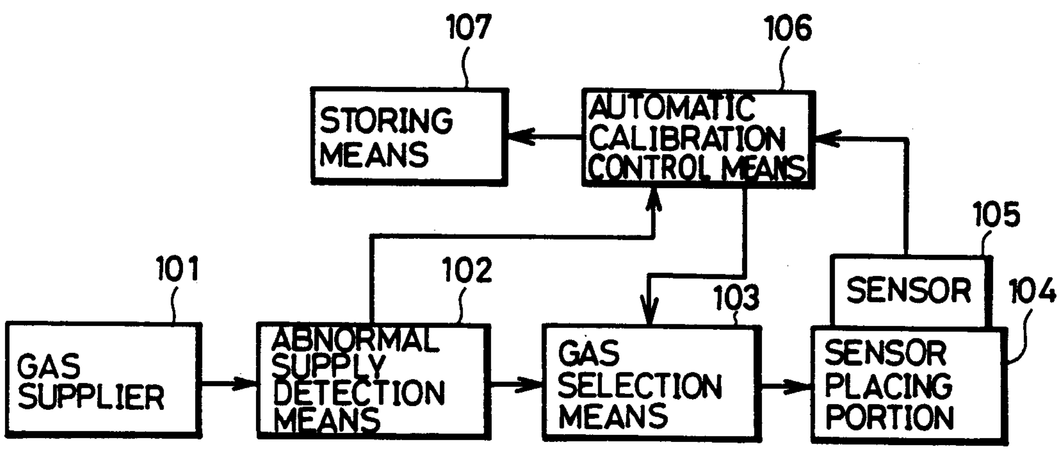
107
106
STORING MEANS
AUTOMATIC CALIBRATION CONTROL MEANS
105
101
102
SENSOR
104
103
GAS SUPPLIER
ABNORMAL SUPPLY DETECTION MEANS
GAS SELECTION MEANS
SENSOR PLACING PORTION

FIG.2

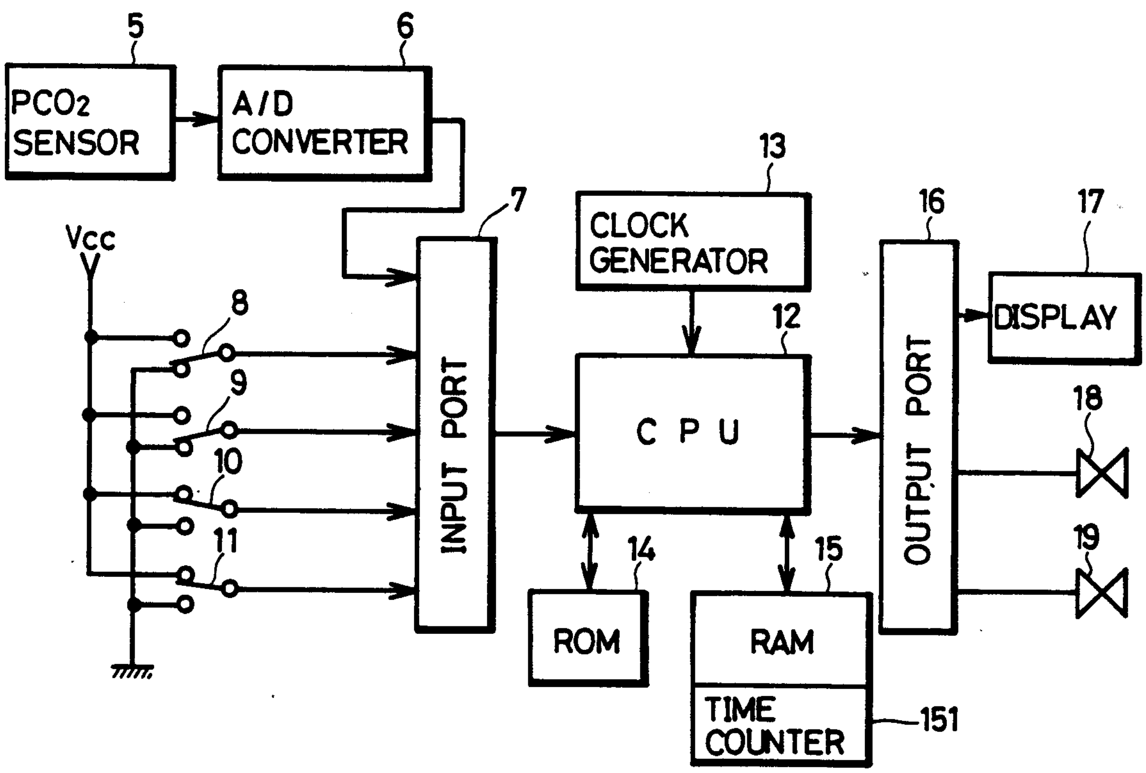
5
6
PCO2 SENSOR
A/D CONVERTER
13
7
CLOCK GENERATOR
16
17
Vcc
8
12
DISPLAY
9
INPUT PORT
C P U
OUTPUT PORT
18
10
11
14
15
19
ROM
RAM
TIME COUNTER
151

FIG.3

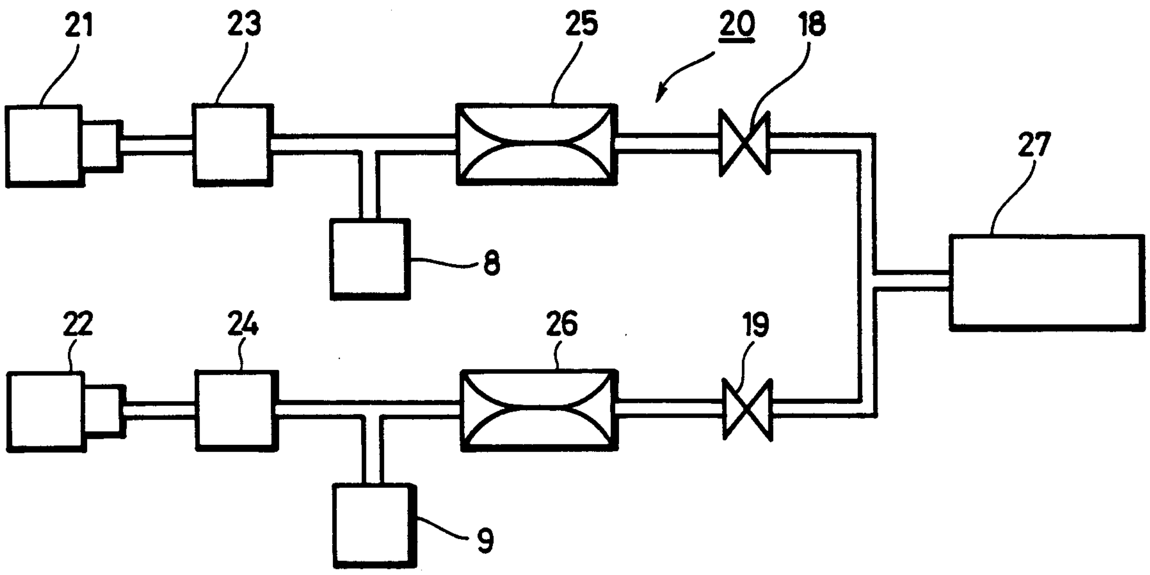


FIG.4

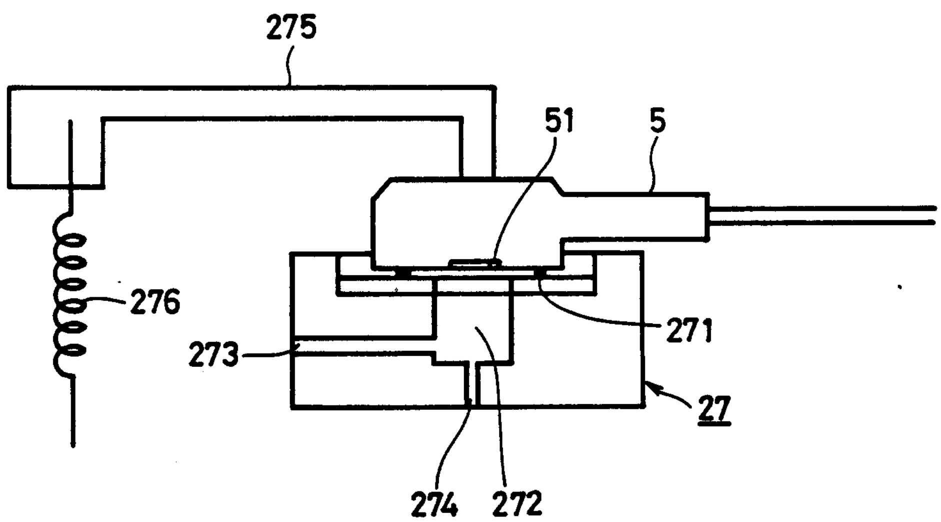

FIG.5

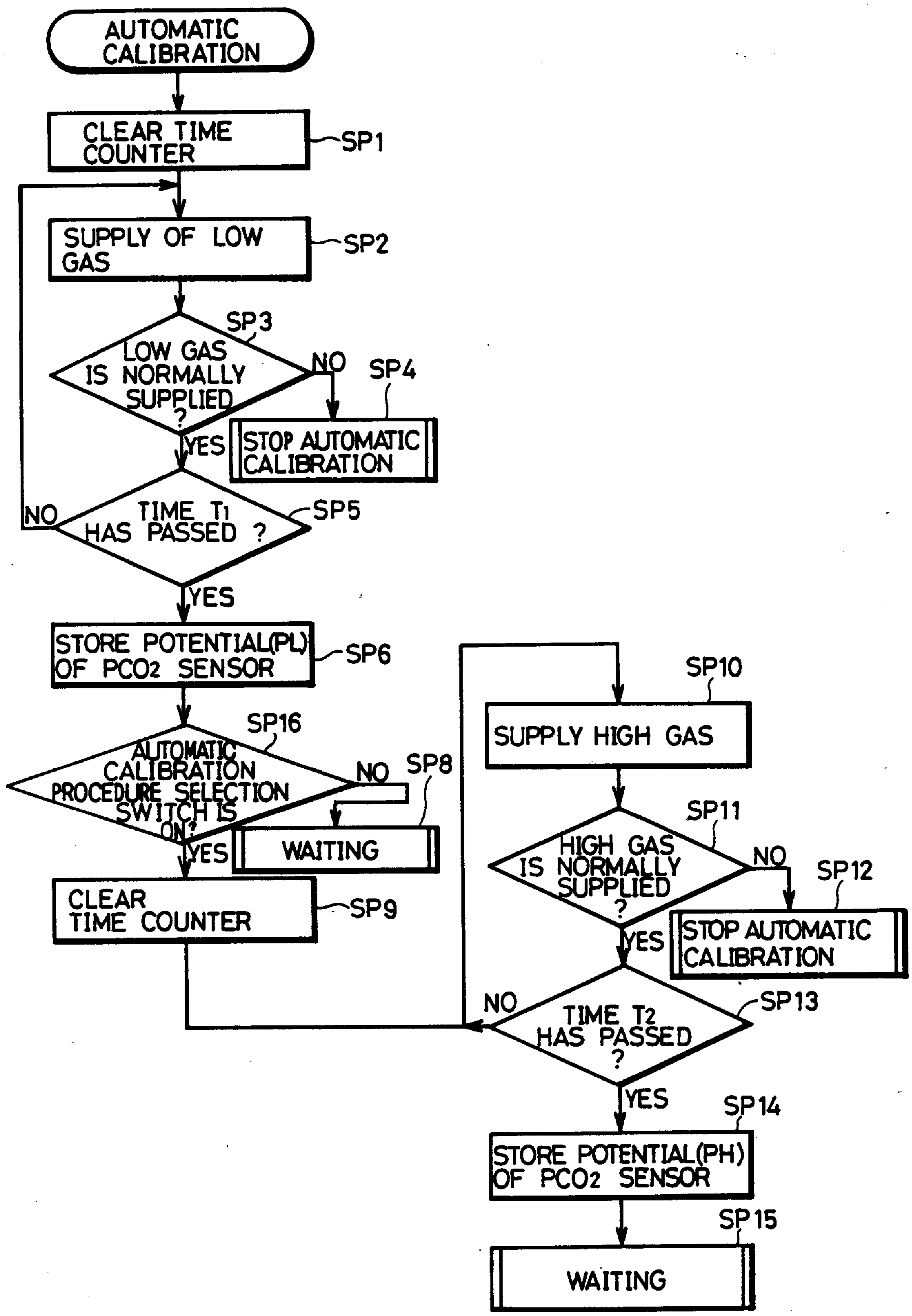
AUTOMATIC CALIBRATION
CLEAR TIME COUNTER
SP1
SUPPLY OF LOW GAS
SP2
LOW GAS IS NORMALLY SUPPLIED ?
SP3
NO
SP4
STOP AUTOMATIC CALIBRATION
YES
TIME T1 HAS PASSED ?
SP5
NO
YES
STORE POTENTIAL(PL) OF PCO2 SENSOR
SP6
AUTOMATIC CALIBRATION PROCEDURE SELECTION SWITCH IS ON?
SP16
NO
SP8
WAITING
YES
CLEAR TIME COUNTER
SP9
SUPPLY HIGH GAS
SP10
HIGH GAS IS NORMALLY SUPPLIED ?
SP11
NO
SP12
STOP AUTOMATIC CALIBRATION
YES
TIME T2 HAS PASSED ?
SP13
NO
YES
STORE POTENTIAL(PH) OF PCO2 SENSOR
SP14
WAITING
SP15

FIG.6

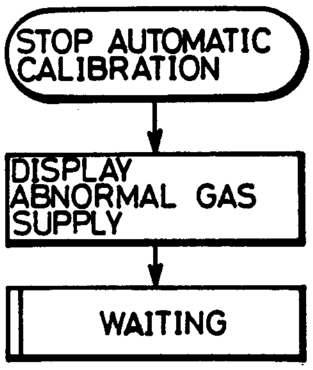


FIG.8

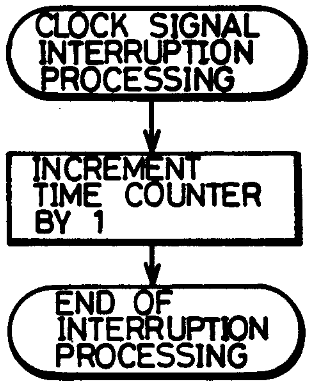


FIG.7

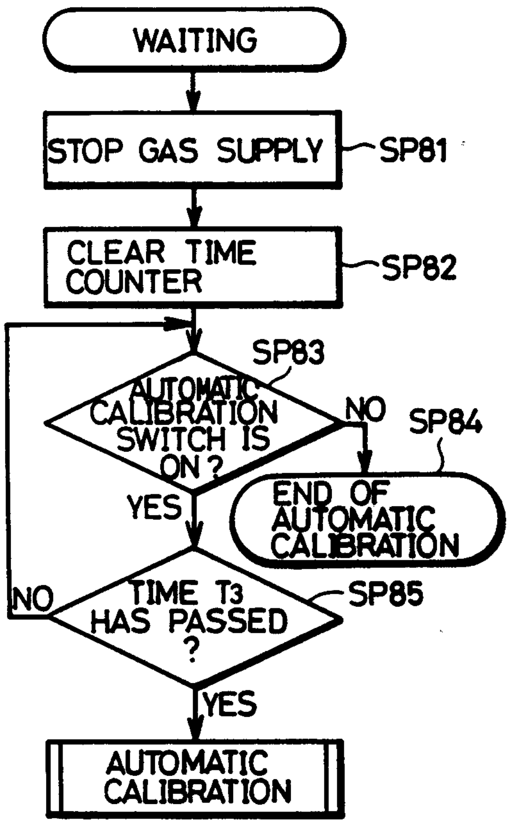


FIG.10

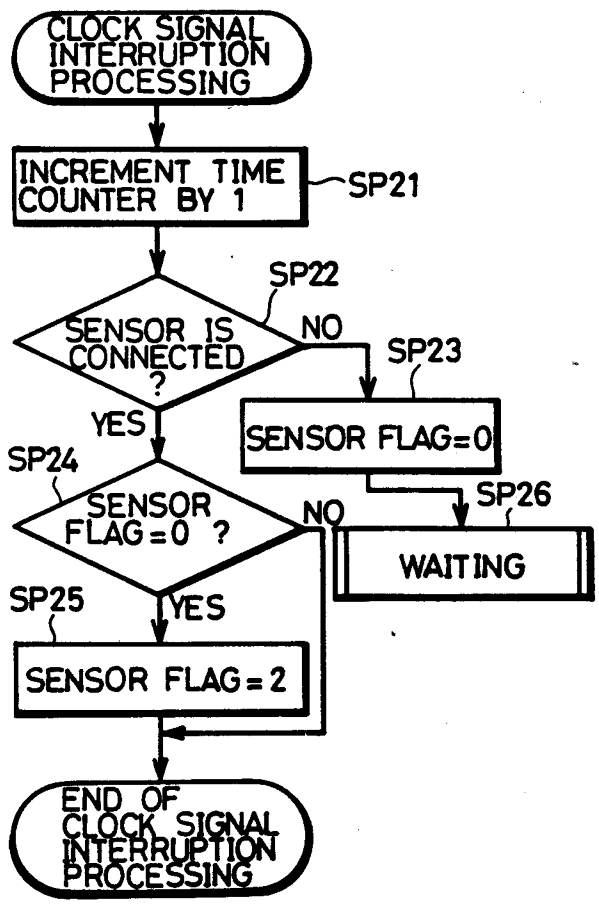

FIG.9A

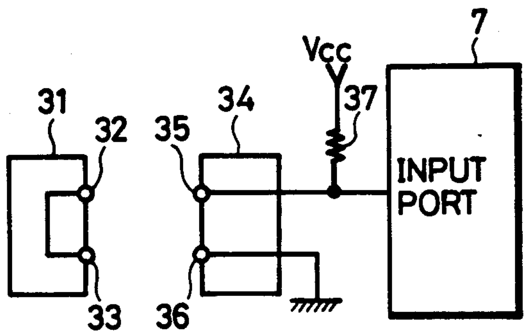


FIG.9B

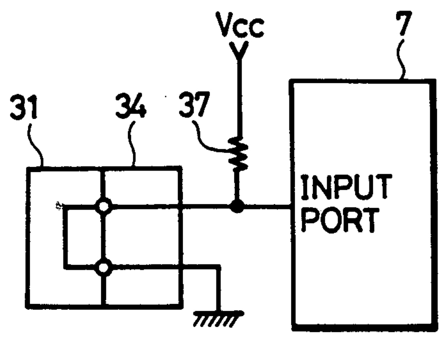


FIG.12

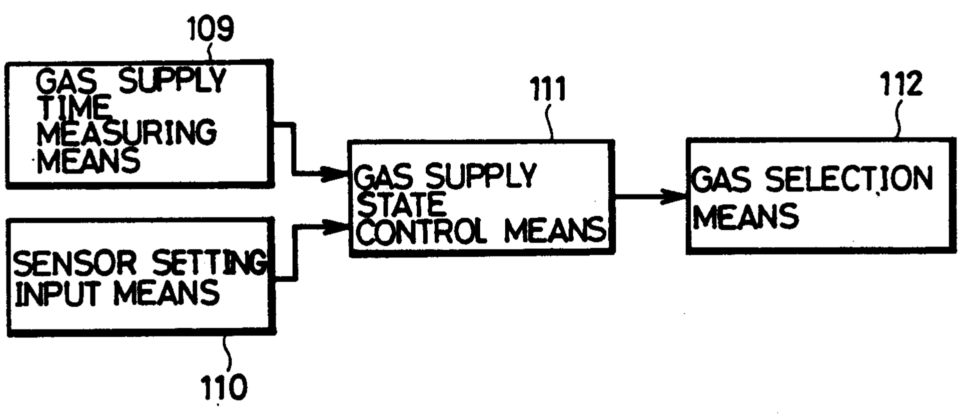

FIG.11

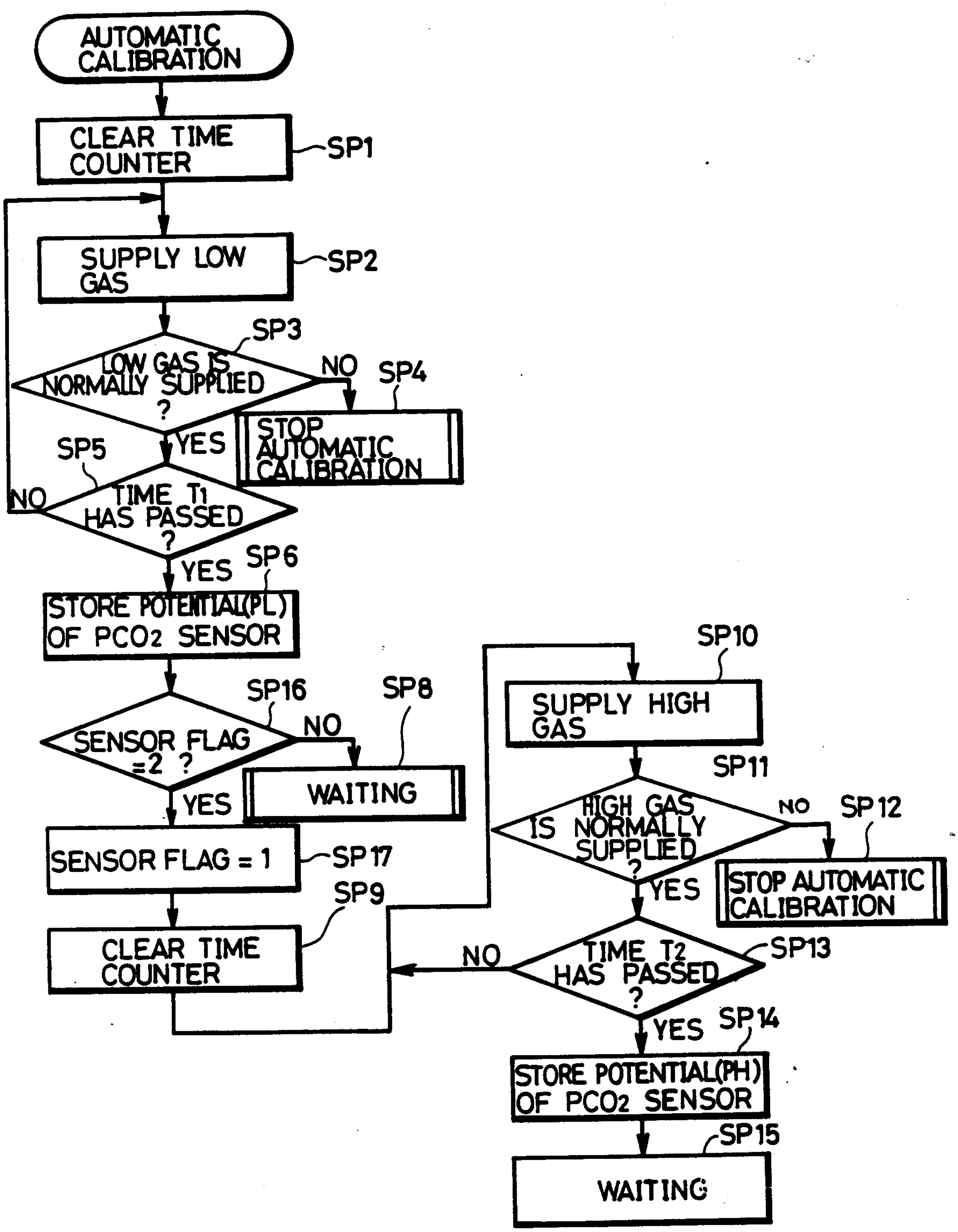
AUTOMATIC CALIBRATION
CLEAR TIME COUNTER
SP1
SUPPLY LOW GAS
SP2
SP3
LOW GAS IS NORMALLY SUPPLIED ?
NO
SP4
STOP AUTOMATIC CALIBRATION
YES
SP5
TIME T1 HAS PASSED ?
NO
YES
SP6
STORE POTENTIAL(PL) OF PCO2 SENSOR
SP16
SP8
SENSOR FLAG =2 ?
NO
WAITING
YES
SENSOR FLAG = 1
SP17
SP9
CLEAR TIME COUNTER
SP10
SUPPLY HIGH GAS
SP11
HIGH GAS IS NORMALLY SUPPLIED ?
NO
SP12
STOP AUTOMATIC CALIBRATION
YES
TIME T2 HAS PASSED ?
SP13
NO
YES
SP14
STORE POTENTIAL(PH) OF PCO2 SENSOR
SP15
WAITING

FIG.13

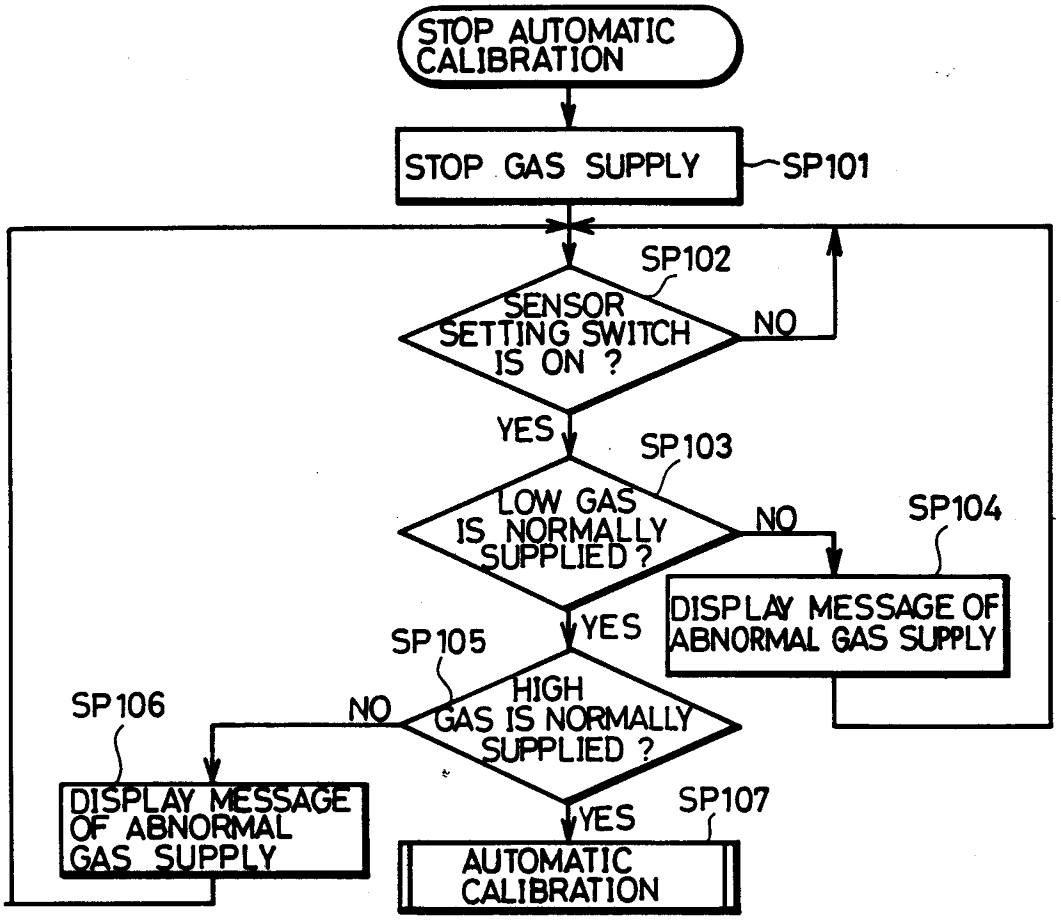
STOP AUTOMATIC CALIBRATION
STOP GAS SUPPLY
SP101
SP102
SENSOR SETTING SWITCH IS ON ?
NO
YES
SP103
LOW GAS IS NORMALLY SUPPLIED ?
NO
SP104
DISPLAY MESSAGE OF ABNORMAL GAS SUPPLY
YES
SP105
HIGH GAS IS NORMALLY SUPPLIED ?
NO
SP106
DISPLAY MESSAGE OF ABNORMAL GAS SUPPLY
YES
SP107
AUTOMATIC CALIBRATION

FIG.15

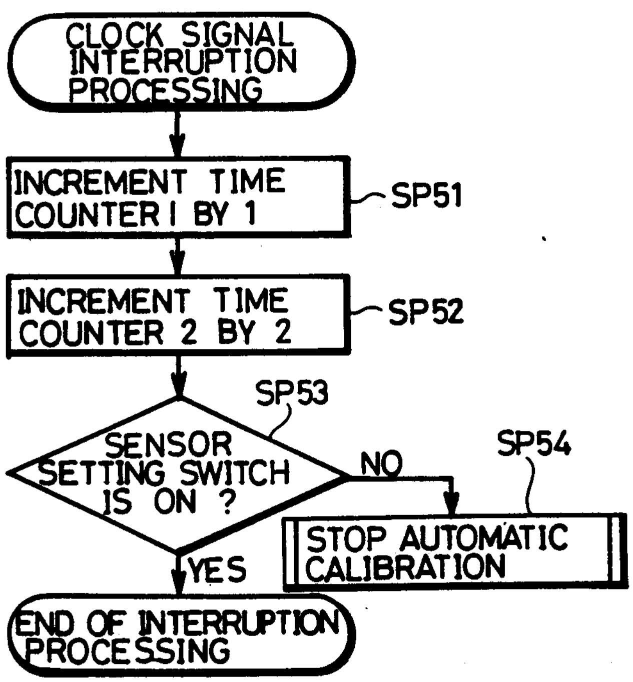
CLOCK SIGNAL INTERRUPTION PROCESSING
INCREMENT TIME COUNTER I BY 1
SP51
INCREMENT TIME COUNTER 2 BY 2
SP52
SP53
SENSOR SETTING SWITCH IS ON ?
NO
SP54
STOP AUTOMATIC CALIBRATION
YES
END OF INTERRUPTION PROCESSING

FIG.14

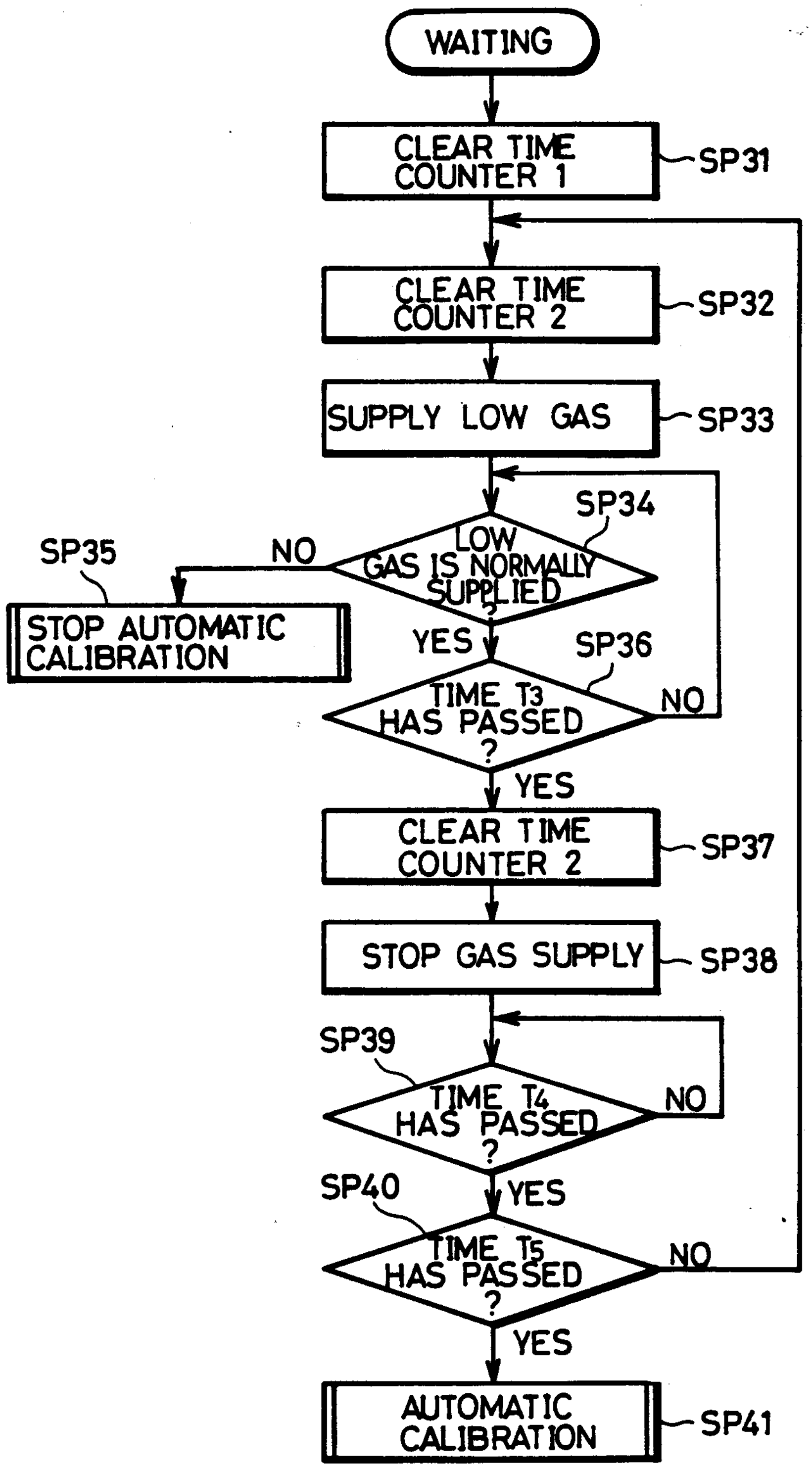
WAITING
CLEAR TIME COUNTER 1
SP31
CLEAR TIME COUNTER 2
SP32
SUPPLY LOW GAS
SP33
SP34
LOW GAS IS NORMALLY SUPPLIED ?
NO
SP35
STOP AUTOMATIC CALIBRATION
YES
SP36
TIME T3 HAS PASSED ?
NO
YES
CLEAR TIME COUNTER 2
SP37
STOP GAS SUPPLY
SP38
SP39
TIME T4 HAS PASSED ?
NO
SP40
YES
TIME T5 HAS PASSED ?
NO
YES
AUTOMATIC CALIBRATION
SP41

FIG.16

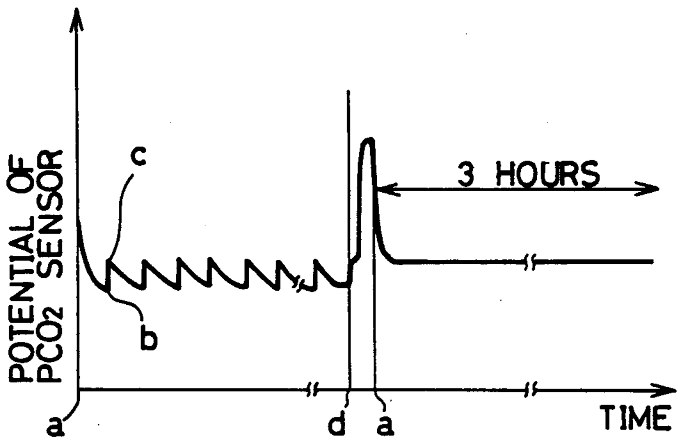


FIG.17

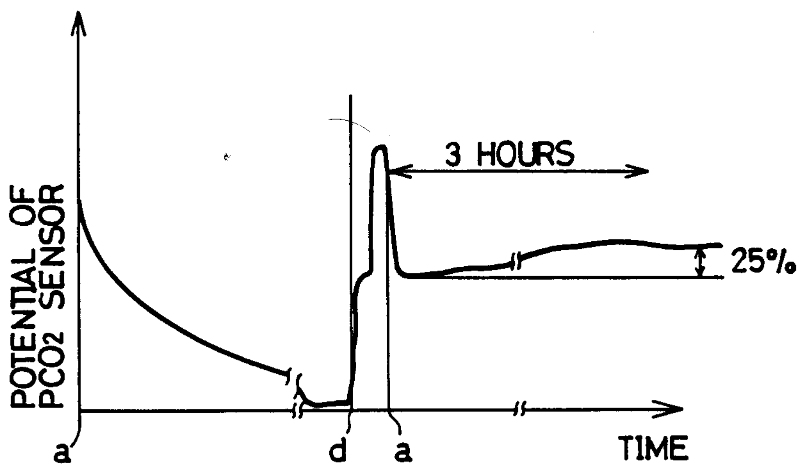


FIG.18